# EUROPÄISCHE PATENTSCHRIFT

(11) **EP 0 304 564 B1**
(45) Veröffentlichungstag und Bekanntmachung des Hinweises auf die Patenterteilung: **05.01.1994**
(21) Anmeldenummer: 88109181.3
(22) Anmeldetag: 09.06.1988
(51) Int. Cl.: A61B 1/26, H05B 41/34, F21S 5/00

(54) **Stroboskoplicht-Generator für insbesondere medizinische Zwecke**
Stroboscopic light generator, in particular for medical purposes
Générateur de lumière stroboscopique, en particulier pour applications médicales

(30) Priorität: 29.07.1987 DE 3725114
(43) Veröffentlichungstag der Anmeldung: 01.03.1989
(73) Patentinhaber: Richard Wolf GmbH, 75438 Knittlingen (DE)
(72) Erfinder: Hoffmann, Reiner, Dipl.-Ing., D-7132 Illingen (DE); Jaggy, Peter, Dipl.-Ing., D-7136 Ötisheim (DE); Vögele, Michael, D-7539 Kämpfelbach/Ersingen (DE); Blanc, Ernst, D-7519 Oberderdingen-Grossvillars (DE)
(74) Vertreter: Wilcken, Thomas, Dipl.-Ing.

(56) Entgegenhaltungen:
- EP-A- 0 066 901
- DE-A- 1 539 349
- DE-A- 2 557 546
- DE-A- 2 849 369
- US-A- 3 274 486
- ICASSP 86 PROCEEDINGS, Tokyo, 7.-11. April 1986, Band 3, Seiten 1633-1636, IEEE; S. KIRITANI et al.: "Simultaneous high-speed digital recording of vocal fold vibration and speech signal"
- Cermax xenon illuminators From: ilc technology, sunnyvale,california,Usa

## Beschreibung

Die Erfindung geht aus von einem Stroboskoplicht-Generator für insbesondere medizinische Zwecke gemäß dem Oberbegriff des Patentanspruches 1. Ein solcher Generator ist in der DE-A-28 49 369 beschrieben.

Derartige Stroboskoplicht-Generatoren kommen im medizinischen Bereich bei der Diagnose von Funktionsstörungen der Stimmlippen zur Anwendung, und zwar vorteilhaft in Verbindung mit einem Laryngoskop, so daß der Arzt einerseits den mit normalen Kaltlicht ausgeleuchteten Kehlkopf und die Stimmlippen in ihrem natürlichen Aussehen und Verhalten beobachten kann und andererseits über eine frequenzsychrone Beleuchtung der Stimmlippen mit Stroboskoplicht die Möglichkeit hat, die durch Erzeugung eines Tones durch den Patienten in Schwingung versetzten Stimmlippen als stehendes Bild zu betrachten. Die relativ kurzen Lichtblitze treffen dabei immer an der gleichen Stelle einer periodischen Bewegungsphase auf die Stimmlippen. Man kann jedoch auch durch gezielte Phasenverschiebung, also durch Verstimmung der Blitzfrequenz, eine verlangsamte Bewegung der Stimmlippen nach Art einer Schwebung darstellen. Die Steuerung der Blitzfrequenz erfolgt bekanntermaßen durch die Schwingungsfrequenz der Stimmlippen.

Weitere, aus dem praktischen Stand der Technik bekannte Stroboskoplicht-Generatoren sind in der Regel so aufgebaut, daß der Lampenteil fest in ein Gerät integriert ist, welches als zusätzliches Beistellgerät zu einem die Steuerschaltung und Steuermittel aufnehmenden Grundgerät zu betreiben ist. Bei dieser Ausführung ist nachteilig, daß ein Lampenwechsel sehr umständlich und ein zusätzlicher Stellplatz erforderlich ist, wobei zudem durch freie Kabelverbindungen zwischen den Geräten eine gewisse Unübersichtlichkeit und Störanfälligkeit gegeben ist. Die bekannten Geräte sind weiter nur für die Stroboskopie einsetzbar, das heißt, daß das im Rahmen der Untersuchung zur Ausleuchtung des Beobachtungsfeldes benötigte Beobachtungslicht mit eine zusätzlichen Lampe erzeugt und deren Dauerlicht über entsprechende optische, mechanische und elektromechanische Mittel in den Strahlengang des Stroboksoplichtes eingebracht wird. Um das Beschlagen des optischen Fensters am distalen Ende des Endoskopes zu vermeiden, wird üblicherweise ein ständiger Luftstrom über dieses geführt oder mit Antibeschlagmitteln gearbeitet, wozu zusätzliche Mittel und Einrichtungen benötigt werden, die wiederum in Form von Zusatzgeräten beigestellt werden. Schließlich wirkt sich die Tatsache aufwanderhöhend aus, daß die Lichtintensität zwischen Beobachtungslicht und Stroboskoplicht mit zunehmender Blitzfrequenz wachsend schwankt, was eine automatische Helligkeitsregelung insbesondere beim Einsatz von Video-Einrichtungen erforderlich macht. Dazu kommt, daß die beiden Lichterzeuger von unterschiedlicher Bauart sind (Blitzlampe, Halogenlampe) und demzufolge Licht unterschiedlicher Farbtemperatur erzeugen, was eine Umschaltung der Kamera erforderlich macht bzw. Bilder mit unterschiedlicher Farbwiedergabe entstehen läßt.

In der DE-A-28 49 369 ist ein Stroboskop-Generator für die Kehlkopfbetrachtung eines Patienten beschrieben, der eine Blitzlichtentladelampe aufweist, die elektronisch so gesteuert wird, um Beobachtungslicht auszusenden, um die Stimmlippen bzw. den Kehlkopfbereich des Patienten betrachten zu können, die aber auch Stroboskoplicht aussendet, wenn die Stimmlippen fotografiert werden sollen, wozu stärkeres Licht erforderlich ist. Zu letzterem wird im Steuerschaltkreis eine Umsteuerung vorgenommen, um die Blitzlichtentladelampe mit einer höheren elektrischen Energie zu versorgen. Die Blitzlichtentladelampe hat eine begrenzte Lebensdauer, so daß sie nach relativ kurzer Benutzungsdauer ausgewechselt werden muß.

Die Aufgabe der Erfindung besteht darin, einen Stroboskopgenerator gemäß der einleitend angeführten Art so zu verbessern, daß Beobachtungslicht sowohl als Dauerlicht als auch als Blitzlichtfolge zur Verfügung steht, daß die sowohl das Beobachtungslicht als auch das Stroboskoplicht erzeugende Lampe schnell und einfach auswechselbar ist, daß auch bei Schonung der Lampe für Beobachtungs- und Stroboskoplicht qualitativ gleiches Beobachtungslicht zur Verfügung steht und daß der Generator reparaturfreundlich ist.

Die Lösung der Aufgabe geht von dem einleitend angeführten Stroboskopgenerator aus und kennzeichnet sich dadurch, daß die Lampe eine Kurzbogenlampe ist, mit der das Beobachtungslicht wahlweise als Dauerlicht oder als eine Folge von Lichtblitzen erzeugbar ist, deren Frequenz einstellbar oder entsprechend einem extern zuführbaren Signal synchronisierbar ist, daß die Kurzbogenlampe in einem Lampeneinschub eingebaut ist, daß zusätzliches Beobachtungslicht mittels einer separaten Lichtquelle mit einem eigenen optischen System erzeugbar ist, welchem eine separate Lichtentnahmestelle zugeordnet ist, daß die Farbtemperaturen des Beobachtungs- und des Stroboskoplichtes annähernd gleich sind und daß sämtliche Bauelemente des Generators in einem Einschubgehäuse untergebracht sind.

Mit der erfindungsgemäßen Lösung ist eine verlängerte Lebensdauer der verwendeten Lampe in Form einer Kurzbogenlampe erreicht, wobei das Beobachtungslicht sowohl als Dauerlicht als auch als eine Folge von Lichtblitzen erzeugt werden kann. Ein späterer Wechsel der Kurzbogenlampe kann infolge ihrer Anordnung in dem Einschub schnell und einfach vorgenommen werden. Dieser Vorteil gilt auch für den erfindungsgemäßen Stroboskop-Generator selbst, da alle seine Bauelemente in einem Einschubgehäuse angeordnet sind, das auf diese Weise von einem größeren Gerät oder einer größeren Einheit leicht abnehmbar ist. Da die Farbtemperaturen des Beobachtungslichtes und des Stroboskoplichtes zumindest annähernd gleich sind, kann eine automatische Helligkeitsregelung entfallen, so daß sich der erfindungsgemäße Generator vorteilhaft für den Einsatz von Video-Einrichtungen zum Aufnehmen von Farbfotos eignet. Ein weiterer Vorteil des Generators besteht darin, daß die empfindliche Kurzbogenlampe geschont werden kann, wenn lediglich eine visuelle Untersuchung vorgenommen wird, bei der eine Ausleuchtung durch einfaches Dauerlicht ausreicht. Es wird dann auf die separate Lichtquelle mit einem eigenen optischen Gesamtsystem umgeschaltet, die das einfache Dauerlicht aussendet. Schließlich ist noch vorteilhaft, daß der erfindungsgemäße Generator auch für andere medizinische Beleuchtungszwecke, bei denen einfaches Dauerlicht ausreicht, verwendet werden kann.
Zwecks Verbesserung der Beobachtungsmöglichkeiten bestimmter zu untersuchender Gegebenheiten kann es von Vorteil sein, wenn die Frequenz der Blitzfolge sowohl des Beobachtungslichtes als auch des Stroboskoplichtes derartig beeinflußbar ist, daß ab einer vorgebbaren Frequenzhöhe, z. B. 440 Hz, eine automatische Frequenzteilung erfolgt.
Um das durch das Zünden der Lampe erzeugte, sich auf den die Lampe aufnehmenden Einschub durch Körperschalleitung übertragende Geräusch zu dämpfen, ist das optische System mit dem die Lampe aufnehmenden Lampeneinschub über Dämpfungselemente miteinander verbunden. Eine konstante Temperatur der Lampe wird dadurch erreicht, daß ein Lüfter zur Kühlung derselben in Abhängigkeit der Lampentemperatur betrieben wird.
Für die Lieferung eines klaren Bildes ist eine Luftpumpe zur Erzeugung eines ständigen für eine Freispülung des distalen Fensters des Endoskopes verwendbaren Spülluftstromes vorgesehen.

Schließlich läßt sich ein leicht handhabbares Gerät dadurch schaffen, daß in der Frontplatte des Einschubgehäuses
die Lichtentnahmestellen in Form von geeigneten Lichtleiter-Anschlußbuchsen und eine Schnellkupplung für die Abnahme des Spülluftstromes angeordnet sind und dessen Rückwand eine Steckverbindung zur Versorgung der Bauelemente mit elektrischer Energie und Einspeisung der Synchronisierungssignale für die Steuerung der Blitzfrequenz der Lampe für das Stroboskoplicht und für das blitzende Beobachtungslicht trägt.

Der erfindungsgemäße Stroboskoplicht-Generator ist nachfolgend anhand eines in der Zeichnung dargestellten Ausführungsbeispiels näher erläutert. In der Zeichnung zeigen:

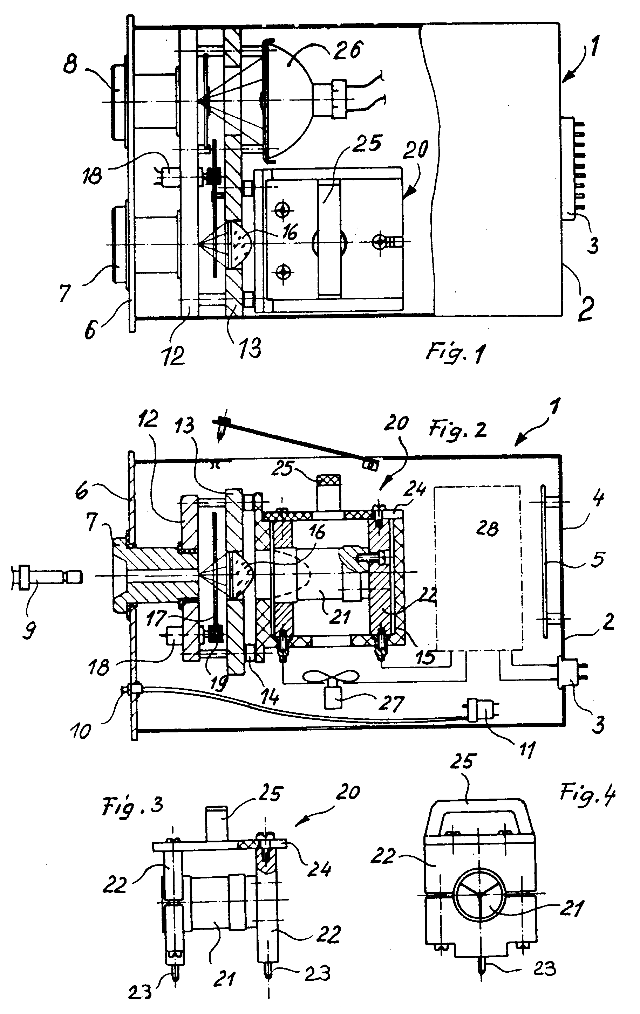

- Figur 1: eine Draufsicht auf den erfindungsgemäßen Stroboskoplicht-Generator unter teilweiser Schnittdarstellung,
- Figur 2: eine Seitenansicht des in Figur 1 gezeigten Generators im Längsschnitt,
- Figur 3: eine Seitenansicht des die Stroboskop-Lampe aufnehmenden Einschubs,

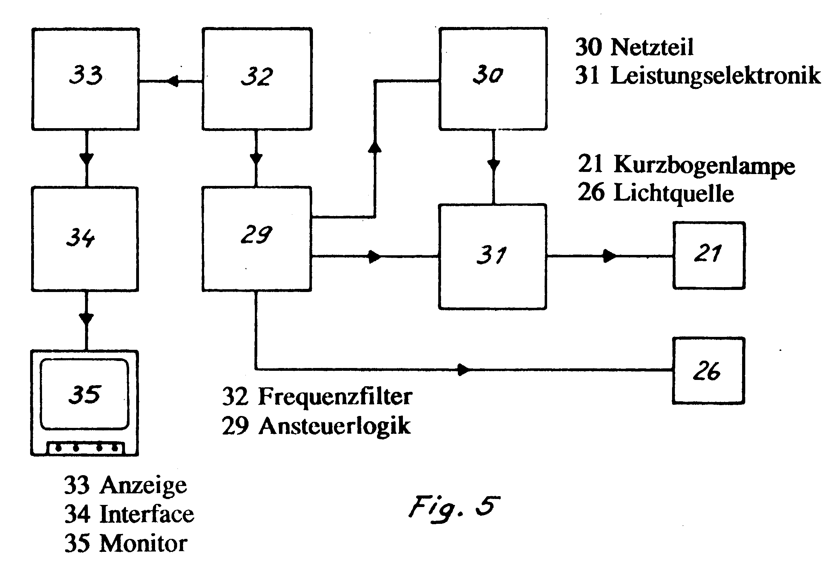

- Figur 4: eine Frontansicht des Einschubs nach Figur 3 und
- Figur 5: ein Blockschaltbild der Elektronikeinheit für den Betrieb des Generators.

Der erfindungsgemäße Stroboskoplicht-Generator ist in einem Einschubgehäuse 1 untergebracht, welches an seiner Rückwand 2 mit einer Steckverbindung 3 in Form einer Steckerleiste versehen ist, die nach Einschieben des Einschubgehäuses 1 in ein nicht gezeigtes Versorgungsgestell oder einen Projektor für die Zuführung der für den Betrieb des Generators erforderlichen elektrischen Energie sowie für dessen Steuerung sorgt. Die Rückwand 2 weist weiter eine Öffnung 4 auf, die durch eine an der Innenfläche mittels Distanzhaltern befestigten Platte 5 unter Belassung eines umlaufenden Lüftungsspaltes abgedeckt ist und dem Luftaustausch mit dem Innenraum des Einschubgehäuses 1 dient.

In der Frontplatte 6 desselben sind zwei Lichtentnahmestellen 7 und 8 zum Steckanschluß eines Lichtleitkabels 9 sowie eine Schnellkupplung 10 zur Abnahme eines Spülluftstromes vorgesehen, der von einer geeigneten Luftpumpe 11 erzeugt wird. Die Lichtentnahmestellen 7 und 8 bestehen jeweils aus einer Lichtleiter-Anschlußbuchse, die sich im Einschubgehäuse in einer Halterung 12 abstützen, die wiederum eine durch Distanzmittel auf Abstand gehaltene Aufnahme 13 trägt. Die Aufnahme 13 ist Träger eines zu der Steckdose der Lichtentnahmestelle 7 koaxial ausgerichteten Lampengehäuses 15, welches mit der Aufnahme 13 durch Dämpfungselemente 14 verbunden ist. Die Aufnahme 13 dient weiter als Fassung für eine Linse 16, die ebenfalls in koaxialer Ausrichtung zu der Steckachse der Lichtentnahmestelle 7 und hinter einer Blende 17 angeordnet ist, die als Sichelblende ausgeführt und mittels eines an der Halterung 12 befestigten Motors 18 über ein Zahnrad 19 verdrehbar ist.

Das Lampengehäuse 15 dient der Aufnahme eines Lampeneinschubs 20 und ist an seiner Konsole mit Buchsen für eine elektrische Steckverbindung für den Betrieb einer in den Lampeneinschub 20 eingesetzten Lampe 21 ausgestattet. Diese ist als mit Gas gefüllte Kurzbogenlampe mit eingebautem Reflektor ausgeführt und besteht aus einem rohrförmigen Lampenkörper, der distal-und proximalseitig in bzw. an Metallkörpern 22 befestigt ist, die mit Spannungszuführungen 23 in Form von in die Buchsen in dem Lampengehäuse 15 einführbaren Steckstiften versehen sind und gleichzeitig der Ableitung der Wärme dienen.

An der oberen Seite des Lampeneinschubs 20 befindet sich eine die beiden Metallkörper 22 brückenartig verbindende Abdeckung 24, an der eine Handhabe 25 zum Entfernen und Einsetzen des Lampeneinschubs 20 befestigt ist. Die Aufnahme 13 ist weiter im Bereich der Lichtentnahmestelle 8 mit einer koaxial zu der Steckachse derselben ausgerichteten zweiten Öffnung versehen und trägt ebenfalls in koaxialer Ausrichtung eine Lichtquelle 26 zur Erzeugung von Dauerlicht mit einem Reflektor, welcher das Licht gebündelt in den Steckanschluß der Lichtentnahmestelle 8 schickt, auf die das Lichtleitkabel 9 bei Bedarf umgesteckt werden kann.

In dem Einschubgehäuse 1 ist weiter ein Lüfter 27 unterhalb des Lampengehäuses 15 eingebaut, welches den Betrieb der Lampe 21 mit einer konstanten Betriebstemperatur ermöglicht, wobei der Zeitpunkt und die Dauer des Betriebes durch die Elektronik 28 gesteuert wird. Das Einschubgehäuse 1 umschließt die der Steuerung des Gerätes dienende Elektronik 28.

Diese umfaßt eine Ansteuerlogik 29, die die extern zugeführten Signale auswertet und je nach Stellung eines nicht gezeigten Fußschalters das Beobachtungslicht oder das Stroboskoplicht aktiviert. Im Falle der Erzeugung beider Lichtarten durch die Lampe 21 wird in einer ersten Stellung des Fußschalters das Beobachtungsdauerlicht durch Ansteuerung eines Netzteils 30 mit großer Leerlaufspannung und stabiler Lastspannung erzeugt, welches über eine Leistungselektronik 31 mit der Lampe 21 verbunden ist, und in einer zweiten Stellung des Fußschalters das mit der Stimmlippenfrequenz synchronisierte Stroboskoplicht durch direkte Ansteuerung der Leistungselektronik 31 erzeugt. Die Lampe 21 wird dabei in den Betriebsstellungen "Beobachtungslicht" und "Stroboskoplicht" durch die gleiche, in die Leistungselektronik 31 integrierte Zündelektronik gezündet. Der Ansteuerlogik 29 ist ein Frequenzfilter 32 vorgeschaltet, welches aus einem von dem Patienten erzeugten Ton das Grundwellensignal herausfiltern kann, um mit diesem Signal die Blitzfrequenz der Lampe 21 bei der Stroboskopie zu triggern. Beim Einsatz der Lampe 21 zur Erzeugung eines blitzenden Beobachtungslichtes wird vorteilhaft eine Blitzfrequenz eingestellt, die unter oder über der Frequenz des Grundwellensignals des von dem Patienten abgenommenen Signals liegt.

Bei Verwendung der separaten Lichtquelle 26 als zusätzliche Möglichkeit zur Erzeugung von Beobachtungslicht erfolgt während des Bedarfs dieser Lichtart eine entsprechende Beschaltung dieser Lichtquelle 26. Die Ansteuerlogik 29 kann weiter eine Anzeigenplantine 33 umfassen, die durch das Frequenzfilter 32 angesteuert wird und dem Anzeigen der Grundwellenfrequenz, die Phase und der Lautstärke des Stimmensignals dient. Diese Daten können durch ein Interface 34 aufbereitet werden, so daß sie auf einem Monitor 35 zur Anzeige gebracht werden können.

## Patentansprüche

1. Stroboskoplicht-Generator für insbesondere medizinische Zwecke mit einer Lichtentnahmestelle (7) zum Anschluß eines flexiblen Lichtleitkabels (9) zur Entnahme von wahlweise einstellbarem, von einer einzigen Lampe (21) erzeugten und über ein optisches System (16,17) zu der erwähnten Stelle (7) gerichteten Stroboskop- und Beobachtungslicht, das über das Lichtleitkabel (9) einem Endoskop zur Untersuchung der Stimmlippenfunktion eines Patienten zuführbar ist, wobei die Frequenz des Stroboskoplichtes variabel einstellbar und insbesondere in Abhängigkeit von der Grundfrequenz des jeweils von den Stimmlippen erzeugten akustischen Signals steuerbar ist, dadurch gekennzeichnet, daß die Lampe (21) eine Kurzbogenlampe ist, mit der das Beobachtungslicht wahlweise als Dauerlicht oder als eine Folge von Lichtblitzen erzeugbar ist, deren Frequenz einstellbar oder entsprechend einem extern zuführbaren Signal synchronisierbar ist, daß die Kurzbogenlampe (21) in einem Lampeneinschub (20) eingebaut ist, daß zusätzliches Beobachtungslicht mittels einer separaten Lichtquelle (26) mit einem eigenen optischen System erzeugbar ist, welchem eine separate Lichtentnahmestelle (8) zugeordnet ist, daß die Farbtemperaturen des Beobachtungs- und des Stroboskoplichtes annähernd gleich sind und daß sämtliche Bauelemente des Generators in einem Einschubgehäuse (1) untergebracht sind.

2. Stroboskoplicht-Generator nach Anspruch 1, dadurch gekennzeichnet, daß die Kurzbogenlampe (21) eine im wesentlichen rohrförmige Entladungslampe ist, die mit den an ihren Enden vorgesehenen elektrischen Anschlüssen in jeweils einem plattenförmigen Metallkörper (22) steckt, und daß die beiden Spannungszuführungen (23) tragende, senkrecht zur Lampenachse stehenden Metallkörper (22) mit einer isolierenden Abdeckung (24) überbrückt sind, die auf ihrer Oberseite eine Handhabe (25) aufweist.

3. Stroboskoplicht-Generator nach Anspruch 1 oder 2, dadurch gekennzeichnet, daß im Strahlengang zwischen der Kurzbogenlampe (21) und der Lichtentnahmestelle (7) eine Blende (17) zur Regelung des Lichtstromes vorgesehen ist.

4. Stroboskoplicht-Generator nach Anspruch 1, dadurch gekennzeichnet, daß die Frequenz der Blitzfolge sowohl des Beobachtungslichtes als auch des Stroboskoplichtes der Kurzbogenlampe (21) derartig beeinflußbar ist, daß ab einer vorgebbaren Frequenzhöhe, z.B. 440 Hz, eine automatische Frequenzteilung erfolgt.

5. Stroboskoplicht-Generator nach Anspruch 1, dadurch gekennzeichnet, daß das optische System (13,16) mit dem die Kurzbogenlampe (21) aufnehmenden Lampeneinschub (20) über Dämpfungselemente (14) miteinander verbunden ist.

6. Stroboskoplicht-Generator nach einem der Ansprüche 1 bis 5, dadurch gekennzeichnet, daß ein Lüfter (27) zur Konstanthaltung der Temperatur der Kurzbogenlampe (21) in Abhängigkeit von deren Temperatur vorgesehen ist.

7. Stroboskoplicht-Generator nach einem der Ansprüche 1 bis 6, dadurch gekennzeichnet, daß eine Luftpumpe (11) zur Erzeugung eines ständigen, für eine Freispülung des distalen Fensters des Endoskopes verwendbaren Spülluftstromes vorgesehen ist.

8. Stroboskoplicht-Generator nach einem der Ansprüche 1 bis 7, dadurch gekennzeichnet, daß in der Frontplatte (6) des Einschubgehäuses (1) die Lichtentnahmestellen (7 und 8) in Form von Lichtleiter-Anschlüssen und eine Schnellkupplung (10) für die Abnahme des Spülluftstromes untergebracht sind und daß die Rückwand (2) des Einschubgehäuses (1) eine Steckverbindung (3) zur Versorgung der Bauelemente des Generators mit elektrischer Energie und Einspeisung der Synchronisierungssignale für die Steuerung der Blitzfrequenz der Kurzbogenlampe (21) trägt.

## Claims

1. A stroboscopic light generator, in particular for medical purposes with a light extraction site (7) for the connection of a flexible photoconducting cable (9) for the extraction of selectively adjustable stroboscopic and observation light, produced by a single lamp (21) and directed via an optical system (16,17) to the above-mentioned site (7), which light is able to be supplied via the photoconducting cable (9) to an endoscope for examination of the vocal chord function of a patient, in which the frequency of the stroboscopic light is variably adjustable and in particular is able to be controlled as a function of the fundamental frequency of the acoustic signal produced at any given time by the vocal chords, characterised in that the lamp (21) is a short arc lamp, with which the observation light is selectively able to be produced as continuous light or as a series of flashes of light, the frequency of which is able to be synchronised adjustably or according to a signal which is able to be supplied externally, that the short arc lamp (21) is incorporated in a lamp insert (20), that additional observation light is able to be produced by means of a separate light source (26) with its own optical system, to which a separate light extraction site (8) is assigned, that the colour temperatures of the observation - and of the stroboscopic light are approximately equal and that all the structural elements of the generator are accommodated in an insert housing (1).

2. A stroboscopic light generator according to Claim 1, characterised in that the short arc lamp (21) is a substantially tubular discharge lamp, which is inserted with the electric connections provided at its ends in each case in a plate-shaped metal body (22), and that the two metal bodies (22), carrying voltage supplies (23) and standing perpendicular to the lamp axis are bridged with an insulating covering (24), which has a handle (25) on its upper side.

3. A stroboscopic light generator according to Claim 1 or 2, characterised in that in the path of rays between the short arc lamp (21) and the light extraction site (7) a shutter (17) is provided to regulate the stream of light.

4. A stroboscopic light generator according to Claim 1, characterised in that the frequency of the series of flashes both of the observation light and also of the stroboscopic light of the short arc lamp (21) is able to be influenced such that from a predeterminable frequency level, e.g. 440 Hz, an automatic frequency division takes place.

5. A stroboscopic light generator according to Claim 1, characterised in that the optical system (13,16) is connected together with the lamp insert (20) holding the short arc lamp (21) via damping elements (14).

6. A stroboscopic light generator according to one of Claims 1 to 5, characterised in that a fan (27) is provided to keep constant the temperature of the short arc lamp (21) as a function of its temperature.

7. A stroboscopic light generator according to one of Claims 1 to 6, characterised in that an air pump (11) is provided for the production of a constant stream of flushing air which is able to be used for a free flushing of the distal window of the endoscope.

8. A stroboscopic light generator according to one of Claims 1 to 7, characterised in that in the front plate (6) of the insert housing (1), the light extraction sites (7 and 8) are accommodated in the form of photoconductor connections and a quick coupling (10) for the removal of the stream of flushing air and that the rear wall (2) of the insert housing (1) carries a plug connection (3) for the supply of the structural elements of the generator with electrical energy and the feeding of the synchronising signals for the control of the flash frequency of the short arc lamp (21).

## Revendications

1. Générateur de lumière stroboscopique, en particulier pour applications médicales, comportant un emplacement de prélèvement de lumière (7), pour le raccordement d'un câble à fibre optique (9) flexible, pour prélever au choix une lumière stroboscopique et d'observation, réglable, produite par une lampe (21) unique et dirigée à l'emplacement (7) mentionné par l'intermédiaire d'un système optique (16, 17), lumière pouvant être amenée, par l'intermédiaire d'un câble à fibre optique (9), à un endoscope, en vue d'examiner le fonctionnement des cordes vocales d'un patient, la fréquence de la lumière stroboscopique étant réglable variable et pouvant en particulier être commandée en fonction de la fréquence de base du signal acoustique spécifique produit par les cordes vocales, caractérisé en ce que la lampe (21) est une lampe à arc court, à l'aide de laquelle la lumière d'observation peut être produite à volonté comme lumière permanente ou sous forme d'une succession d'éclairs lumineux, dont la fréquence est réglable ou peut être synchronisée de manière correspondante à un signal pouvant être fourni de l'extérieur, en ce que la lampe à arc court (21) est incorporée dans un embout à lampe (20), en ce que de la lumière d'observation supplémentaire peut être produite au moyen d'une source lumineuse (26) séparée, avec un système optique propre, auquel est associé un emplacement de prélèvement de lumière (8) séparé, en ce que les températures de couleur de la lumière d'observation et de la lumière stroboscopique sont à peu près égales et en ce que l'ensemble des éléments de construction du générateur sont logés dans un carter à enfichage (1).

2. Générateur de lumière stroboscopique selon la revendication 1, caractérisé en ce que la lampe à arc court (21) est une lampe à décharge sensiblement tubulaire, s'enfichant, avec les raccordements électriques prévus à ses extrémités, chaque fois dans un corps métallique (22) en forme de plaque, et en ce que des corps métalliques (22) placés perpendiculairement par rapport à l'axe de lampe et portant les deux amenées de tension (23), sont pontés par un recouvrement isolant (24), présentant une poignée (25) sur son côté supérieur.

3. Générateur de lumière stroboscopique selon la revendication 1 ou 2, caractérisé en ce qu'un diaphragme (17) destiné à régler le flux lumineux est prévu dans le trajet du rayon, entre la lampe à arc court (21) et l'emplacement de prélèvement de lumière (7).

4. Générateur de lumière stroboscopique selon la revendication 1, caractérisé en ce que la fréquence de la suite d'éclairs, tant de la lumière d'observation qu'également de la lumière stroboscopique de la lampe à arc court (21) peut être influencée de manière à ce qu'une division de fréquence automatique s'effectue à partir d'une hauteur de fréquence pouvant être allouée, par exemple de 440 Hz.

5. Générateur de lumière stroboscopique selon la revendication 1, caractérisé en ce que le système optique (13, 16) est relié, par l'intermédiaire d'éléments amortisseurs (14), à l'embout de lampe (20) recevant la lampe à arc court (21).

6. Générateur de lumière stroboscopique selon l'une des revendications 1 à 5, caractérisé en ce qu'est prévu un ventilateur (27) destiné au maintien constant de la température de la lampe à arc court (21) en fonction de sa température.

7. Générateur de lumière stroboscopique selon l'une des revendications 1 à 6, caractérisé en ce qu'est prévue une pompe à air (11), destinée à produire un courant d'air de balayage permanent, utilisable pour dégager par balayage la fenêtre distale de l'endoscope.

8. Générateur de lumière stroboscopique selon l'une des revendications 1 à 7, caractérisé en ce que dans la plaque avant (6) du boitier à enfichage (1), sont logés les emplacements de prélèvement de lumière (7 et 8), sous la forme de raccordements de fibres optiques, et un raccord rapide (10) pour le prélèvement du courant d'air de balayage et en ce que la paroi arrière (2) du boîtier à enfichage (1) porte une liaison à enfichage (3) pour l'alimentation des composants du générateur avec de l'énergie électrique et l'introduction des signaux de synchronisation pour la commande de la fréquence d'éclair de la lampe à arc court (21).
